# EUROPEAN PATENT APPLICATION

(11) **EP 4 667 372 A1**
(43) Date of publication of application: **24.12.2025**
(21) Application number: 25174272.2
(22) Date of filing: 05.05.2025
(51) Int. Cl.: B65B 25/14, B65B 61/02, B65B 61/18, B65B 5/06, B65B 11/00, B65B 11/10, B65B 11/12, B65B 11/58, B65D 85/07, B65D 71/06, B65B 63/02, B65D 75/12

(54) **A PACKAGE OF ABSORBENT SANITARY ARTICLES, METHOD AND MACHINE FOR PACKAGING ABSORBENT SANITARY ARTICLES**

(30) Priority: 14.05.2024 IT 202400010822
(71) Applicant: Fameccanica.Data S.p.A., 66020 San Giovanni Teatino (CH) (IT)
(72) Inventor: SABLONE, Gabriele, I-66020 San Giovanni Teatino (Chieti) (IT)
(74) Representative: Buzzi, Notaro & Antonielli d'Oulx S.p.A.

(57) **Abstract**

A package of absorbent sanitary articles, comprising
- a pack (10) formed by a plurality of absorbent sanitary articles (12) having respective main faces (14) parallel to each other and parallel to a longitudinal direction (A),
- a packaging sheet (20) wrapped around the longitudinal faces (16) of the pack (10) and folded to form trapezoid closure flaps (22) extending over the transverse faces (18) of the pack (10), and
- a closure sheet (24) wrapped around the pack (10), wherein the closure sheet (24) has two opposite longitudinal sections (26) extending over two opposite longitudinal faces (16) of the pack (10) and two opposite transverse sections (28) extending over the respective transverse faces (18) above the respective closure flaps (22).

## Description

### Field of the Invention

The present invention relates to a package of absorbent sanitary articles.

The invention has been developed particularly for the packaging of feminine hygiene products such as panty liners, light incontinence pads, sanitary napkins, or similar items.

In the following description, reference will be made to this specific field of application without, however, losing generality.

According to another aspect, the invention relates to a method and a machine for packaging absorbent sanitary articles.

### Description of the Related Art

Absorbent sanitary articles with a thin shape, such as feminine hygiene products, are often individually packaged in respective pouches. In some cases, each pouch may contain two or more absorbent sanitary articles.

The pouches can be made from sheets of plastic material, such as polyethylene, or paper material.

Absorbent sanitary articles packaged in pouches have a flat and thin shape with two main opposing faces. At the exit of a production line, the pouches containing the absorbent sanitary articles are collected into packs, each made up of a predetermined number of pouches aligned in a direction orthogonal to the main faces of the pouches.

Traditionally, the packs made up of a predetermined number of pouches containing absorbent sanitary articles are packaged in a film of plastic material. The most common solution involves using pre-formed plastic bags, which are fed into a packaging machine that inserts the packs of pouches into the bags and seals the plastic bags using heat sealing.

In recent years, there has been growing interest in reducing the amount of plastic waste. However, in the field of packaging absorbent sanitary articles, no alternatives to the use of thermoplastic wrapping have been proposed so far, partly due to the difficulty in packaging compressed and irregularly shaped packs using non-flexible packaging materials.

EP-A-0618148 describes a package for absorbent sanitary articles comprising a pack of absorbent sanitary articles that is kept in a compressed condition by an inner paper wrapper having a tubular shape with a rectangular cross-section that extends around the pack, with its longitudinal axis orthogonal to the pack-forming direction.

The pack enclosed in the inner paper wrapper is contained within an outer thermoplastic wrapper sealed by heat sealing.

GB-A-911273 describes a packaging machine in which a pack of magazines is first wrapped with a band that is secured with an adhesive element, and subsequently wrapped with a packaging sheet folded around the pack. The folded flaps of the packaging sheet are secured with an adhesive strip.

Whether the wrapper is made from a plastic sheet sealed by heat sealing or from a paper sheet folded and sealed with adhesive, the wrapper has two or more faces featuring closure formations, such as welded sheet portions or folded flaps.

On the faces where closure formations are present, it is generally not possible to display graphic information such as logos, images, barcodes, text, etc.

The closure formations thus limit the useful surfaces for commercial communication of the product.

### Object and Summary of the Invention

The object of the present invention is to provide a package for absorbent sanitary articles that overcomes the problems of the prior art.

According to the present invention, this object is achieved by a package having the features set forth in claim 1.

According to another aspect, the invention relates to a method and a machine for packaging absorbent sanitary articles having the features set forth in claims 6 and 10.

### Brief Description of the Drawings

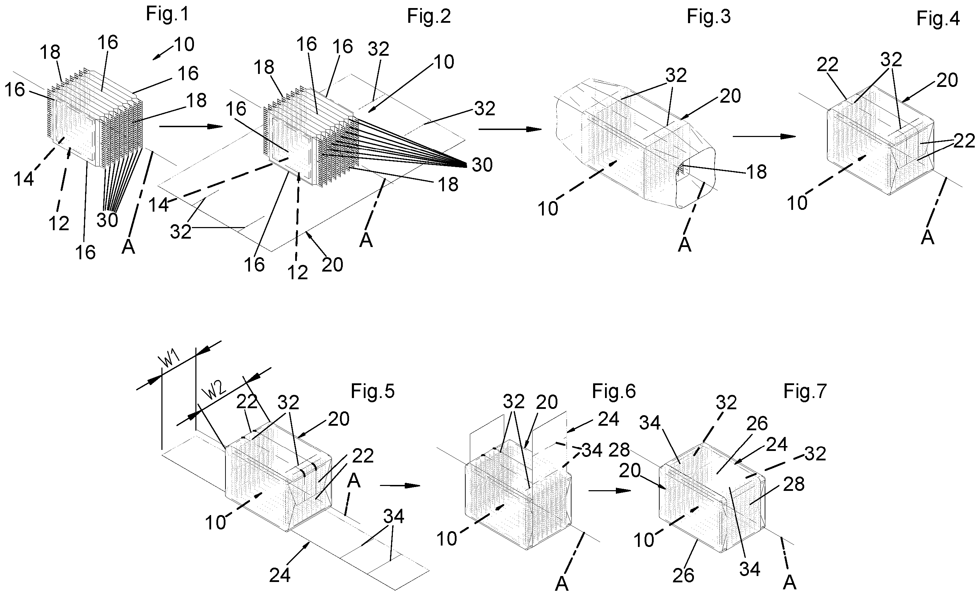

The present invention will now be described in detail with reference to the accompanying drawings, provided by way of non-limiting example, in which Figures 1-7 schematically illustrate the stages of a method for producing packages of absorbent sanitary articles according to the present invention.

### Detailed Description

Figures 1-7 schematically illustrate a method for producing packages of absorbent sanitary articles.

In a first step, the method involves forming a pack 10 consisting of a plurality of absorbent sanitary articles 12. The absorbent sanitary articles 12 may be feminine hygiene products such as panty liners, light incontinence pads, sanitary pads, or similar items. Each absorbent sanitary article 12 has a generally flat shape with two main faces 14 parallel to each other.

In the pack 10, the absorbent sanitary articles 12 are placed adjacent to each other with their respective main faces 14 in contact. Within the pack 10, the main faces 14 of the absorbent sanitary articles 12 are parallel to a longitudinal direction A. The longitudinal direction A is orthogonal to a direction in which the absorbent sanitary articles 12 are placed or stacked to form the pack 10.

In a possible embodiment, the absorbent sanitary articles 12 may be enclosed in respective pouches 30. Each pouch 30 may contain a single absorbent sanitary article 12 or two or more absorbent sanitary articles 12.

The pack 10 has a generally parallelepiped shape with four longitudinal faces 16 parallel to the longitudinal direction A and two transverse faces 18 orthogonal to the longitudinal direction A. The longitudinal faces 16 and transverse faces 18 refer to flat surfaces tangent to the respective edges of the absorbent sanitary articles 12 or the edges of the pouches 30 containing the absorbent sanitary articles 12.

With reference to Figures 2 and 3, the pack 10 is enclosed in a packaging sheet 20. The packaging sheet 20 is initially wrapped around the longitudinal faces 16 of the pack 10. Wrapping the packaging sheet 20 around the longitudinal faces 16 compresses the absorbent sanitary articles 12 together in the direction in which they are placed or stacked.

Then, as shown in Figures 3 and 4, lateral portions of the packaging sheet 20 that protrude beyond the transverse faces 18 of the pack 10 are folded to form trapezoid closure flaps 22 extending over the transverse faces 18 of the pack 10.

The operations of wrapping and folding the packaging sheet 20 around the pack 10 can be carried out using a folding unit with a structure and function known in the packaging field.

The packaging sheet 20 may be made of paper material or biodegradable and compostable plastic material, such as Mater-Bi^{®}. In possible embodiments, the packaging sheet 20 may be made entirely or partially of transparent material, such as cellophane.

With reference to Figure 2, in a preferred embodiment, first tear lines 32 are formed on the packaging sheet 20 before wrapping it around the pack 10. The tear lines 32 are made with cuts that facilitate tearing the packaging sheet 20. After wrapping, the tear lines 32 extend on one of the longitudinal faces 16 of the pack 10 and are transverse to the longitudinal direction A.

With reference to Figures 5 and 6, after the pack 10 has been wrapped in the packaging sheet 20 as shown in Figure 4, a closure sheet 24 is wrapped around the pack 10.

The closure sheet 24 is wrapped around the pack 10 using a folding unit with a known structure and function in the packaging field.

With reference to Figure 7, after the closure sheet 24 is wrapped around the pack 10, it has two opposing longitudinal sections 26 extending over two opposing longitudinal faces 16 and two opposing transverse sections 28 extending over the transverse faces 18, covering the closure flaps 22.

The opposite ends of the closure sheet 24 can be secured together with glue. The closure flaps 22 are held in their folded configuration between the transverse faces 18 of the pack 10 and the transverse sections 28 of the closure sheet 24. Thus, the closure sheet 24 holds the packaging sheet 20 around the pack 10 without needing glue to secure the closure flaps 22.

The closure sheet 24 has a width W1 smaller than the width W2 of the pack 10 in a direction orthogonal to the longitudinal direction A, so the lateral edges of the closure sheet 24 do not protrude beyond the lateral edges of the respective longitudinal faces 16.

The closure sheet 24 can be made of paper material or biodegradable and compostable plastic material, such as Mater-Bi^{®}.

In a possible embodiment, second tear lines 34 can be formed on the closure sheet 24 before wrapping it around the pack 10. After wrapping, the second tear lines 34 are parallel to the first tear lines 32 and may overlap them. Like the first tear lines 32, the second tear lines 34 facilitate tearing the closure sheet 24.

A machine for packaging sanitary articles configured to implement the packaging method described may include compression elements for compressing the pack, a first wrapping unit for wrapping the packaging sheet 20 around the pack 10, side folders for folding the lateral portions of the packaging sheet 20 to form trapezoid closure flaps 22, and a second folding unit for wrapping the closure sheet 24 around the pack 10.

Figure 7 shows a package 36 obtained with the described packaging method. The package 36 has a generally parallelepiped shape where two of the outer surfaces are formed by two longitudinal faces 16 of the packaging sheet 20, and the remaining surfaces are partially covered by two longitudinal sections 26 and two transverse sections 28 of the closure sheet 24.

In possible embodiments, the package 36 may have transparent areas allowing visibility of the products inside. Specifically, the packaging sheet 20 may have transparent portions, such as cellophane, facing the longitudinal faces 16 not covered by the longitudinal sections 26 of the closure sheet 24.

One of the main advantages of the package 36 is that no closure formations disrupt images or text on the outer surfaces. Therefore, all external surfaces of the package 36 can be used for graphic information such as logos, images, barcodes, text, etc.

Thus, all faces of the package 36, including those with trapezoid closure flaps, can be used for commercial communication.

The package 36 has a clean design that conveys order and quality, more effective than conventional packaging for commercial information.

With the same number and size of absorbent sanitary articles, the package is more compact. The absorbent sanitary articles are oriented for compression during wrapping.

Finished packages have regular shapes and sizes, simplifying shipment and shelf arrangement.

Glue use is minimized, as the closure sheet 24 can hold the packaging sheet 20 closed.

The closure sheet width remains unchanged for varying product counts, simplifying format changes.

Naturally, without prejudice to the principle of the invention, the construction details and embodiments may be widely varied without departing from the scope of the invention as defined by the following claims.

## Claims

1. A package of absorbent sanitary articles comprising:
- a pack (10) formed by a plurality of absorbent sanitary articles (12) having respective main faces (14) parallel to each other and parallel to a longitudinal direction (A), wherein said pack (10) has four longitudinal faces (16) parallel to said longitudinal direction (A) and two transverse faces (18) orthogonal to said longitudinal direction (A),
- a packaging sheet (20) wrapped around said longitudinal faces (16) of the pack (10) and folded to form trapezoid closure flaps (22) extending over said transverse faces (18), and
- a closure sheet (24) wrapped around said pack (10) enclosed in said packaging sheet (20), wherein the closure sheet (24) has two opposite longitudinal sections (26) extending over two opposite longitudinal faces (16) of the pack (10) and two opposite transverse sections (28) extending over respective transverse faces (18) above the respective closure flaps (22).

2. A package according to claim 1, wherein said absorbent sanitary articles (12) are enclosed in respective pouches (30).

3. A package according to claim 1 or claim 2, wherein said packaging sheet (20) has transparent areas through which the products contained in the package (36) can be seen.

4. A package according to any of the preceding claims, wherein said packaging sheet (20) has two first tear lines (32) extending over one of said longitudinal faces (16), wherein said first tear lines (32) are parallel to each other and transverse to said longitudinal direction (A).

5. A package according to claim 4, wherein said closure sheet (24) has two second tear lines (34) parallel to respective first tear lines (32).

6. A method for packaging absorbent sanitary articles, comprising:
- forming a pack (10) including a plurality of absorbent sanitary articles (12) having respective main faces (14) parallel to each other and parallel to a longitudinal direction (A), wherein said pack (10) has four longitudinal faces (16) parallel to said longitudinal direction (A) and two transverse faces (18) orthogonal to said longitudinal direction (A),
- wrapping a packaging sheet (20) around said longitudinal faces (16) of the pack (10) and folding said packaging sheet (20) to form trapezoid closure flaps (22) extending over said transverse faces (18), and
- wrapping a closure sheet (24) around said pack (10) enclosed in said packaging sheet (20), wherein the closure sheet (24) has two opposite longitudinal sections (26) extending over two opposite longitudinal faces (16) of the pack (10) and two opposite transverse sections (28) extending over respective transverse faces (18) above the respective closure flaps (22).

7. A method according to claim 6, wherein said absorbent sanitary articles (12) are enclosed in respective pouches (30) before forming said pack (10).

8. A method according to claim 6 or claim 7, comprising forming first tear lines (32) parallel to each other on said packaging sheet (20) before wrapping said packaging sheet (20) around said pack (10), wherein after wrapping the packaging sheet (20) around the pack (10), said first tear lines (32) extend over one of said longitudinal faces (16) and are transverse to said longitudinal direction (A).

9. A method according to claim 8, comprising forming second tear lines (34) parallel to each other on said closure sheet (24) before wrapping said closure sheet (24) around said pack (10) enclosed in said packaging sheet (20), wherein after wrapping the closure sheet (24) around said pack (10) enclosed in said packaging sheet (20), said second tear lines (34) are parallel to respective first tear lines (32).

10. A machine for packaging absorbent sanitary articles, configured to implement a method according to any of claims 6-9.
